# EUROPÄISCHE PATENTSCHRIFT

(11) **EP 3 233 033 B1**
(45) Veröffentlichungstag und Bekanntmachung des Hinweises auf die Patenterteilung: **17.06.2020**
(21) Anmeldenummer: 15801358.1
(22) Anmeldetag: 17.11.2015
(51) Int. Cl.: A61Q 5/04, A61Q 5/10, A61K 8/34, A61K 8/41, A61K 8/49

(54) **RESORCINDERIVATE IN MITTELN ZUM GLEICHZEITIGEN UMFORMEN UND FÄRBEN VON KERATINISCHEN FASERN**
RESORCINOL DERIVATIVES IN COMPOSITIONS FOR SIMULTANEOUS RESHAPING AND COLOURING OF KERATINOUS FIBRES
DÉRIVÉS DE RÉSORCINE DANS DES PRODUITS DE TRANSFORMATION ET DE COLORATION SIMULTANÉE DE FIBRES KÉRATINIQUES

(30) Priorität: 17.12.2014 DE 102014226320
(43) Veröffentlichungstag der Anmeldung: 25.10.2017
(73) Patentinhaber: Henkel AG & Co. KGaA, 40589 Düsseldorf (DE)
(72) Erfinder: GEBERT-SCHWARZWÄLDER, Antje, 41469 Neuss (DE); KOENEN, Annika, 41516 Grevenbroich (DE)
(86) Internationale Anmeldenummer: PCT/EP2015/076790
(87) Internationale Veröffentlichungsnummer: WO 2016/096283

(56) Entgegenhaltungen:
- EP-A1- 0 310 675
- EP-A1- 1 743 620
- EP-A2- 1 287 812
- WO-A2-2011/082910
- WO-A2-2012/038114
- US-A- 5 196 189

## Beschreibung

Die vorliegende Erfindung liegt auf dem Gebiet der Kosmetik und betrifft ein Verfahren zum gleichzeitigen Umformen und Färben von keratinischen Fasern, insbesondere menschlichen Haaren, das die Herstellung eines Mittels durch Vermischen von zwei getrennt voneinander konfektionierten Zubereitungen (A) und (B) umfasst. Die erste Zubereitung (A) beinhaltet ein oder mehrere Resorcinderivate und Oxidationsfarbstoffvorprodukte vom Entwicklertyp und ist durch einen molaren Überschuss an Resorcinderivaten gekennzeichnet. Die zweite Zubereitung (B) enthält Wasserstoffperoxid. Ein zweiter Gegenstand der vorliegenden Erfindung ist die Verwendung dieses derart hergestellten Mittels zum Umformen und Färben von menschlichen Haaren. Für die Erzeugung von Locken oder zur Glättung seiner Haare greift der Anwender zu kosmetischen Umformungsmitteln. Eine dauerhafte Umformung keratinischer Fasern wird üblicherweise derart durchgeführt, dass die Faser mechanisch verformt und die Verformung durch geeignete Hilfsmittel festlegt wird. Vor, während oder nach dieser Verformung behandelt man die Faser mit einer Keratin reduzierenden Zubereitung. Nach einem Spülvorgang wird die Faser dann in dem so genannten Fixierschritt mit einer Oxidationsmittelzubereitung behandelt, gespült und nach oder während des Fixierschritts von den Verformungshilfsmitteln (z.B. Wicklern, Papilloten) befreit. Wenn als Keratin reduzierende Komponente ein Merkaptan, z.B. Ammoniumthioglykolat, verwendet wird, spaltet dieses einen Teil der Disulfid-Brücken des Keratin-Moleküls zu -SH-Gruppen, so dass es zu einer Erweichung der Keratinfaser kommt. Bei der späteren oxidativen Fixierung werden erneut Disulfid-Brücken im Haarkeratin geknüpft, so dass das Keratingefüge in der vorgegebenen Verformung fixiert wird. Alternativ ist es bekannt, zur Haarverformung anstelle der Merkaptane Sulfit zu verwenden. Durch Hydrogensulfit-Lösungen und/oder Sulfit-Lösungen und/oder Disulfit-Lösungen werden Disulfid-Brücken des Keratins in einer Sulfitolyse nach der Gleichung

R - S - S - R + HSO₃⁽⁻⁾ → R-SH + R - S - SO₃⁽⁻⁾

gespalten und auf diese Weise eine Erweichung der Keratinfaser erreicht. Hydrogensulfit-, sulfit- bzw. disulfithaltige Reduktionsmittel weisen nicht den starken Eigengeruch der merkaptanhaltigen Mittel auf. Die Spaltung kann wie zuvor geschildert in einem Fixierschritt mit Hilfe eines Oxidationsmittels unter Bildung von neuen Disulfid-Brücken wieder rückgängig gemacht werden.

Die permanente Glättung keratinhaltiger Fasern wird analog durch den Einsatz von Keratin reduzierenden und -oxidierenden Zusammensetzungen erzielt. In einem entsprechenden Verfahren wird das krause Haar entweder auf Wickler mit einem großen Durchmesser von üblicherweise mehr als 15 mm gewickelt oder das Haar unter Einwirkung der Keratin reduzierenden Zusammensetzung glattgekämmt. Anstelle des Wicklers ist es auch möglich, die Faser auf ein Glättungsboard glattzulegen oder das Haar während des Glättungsprozesses mit einem Kamm glatt zu ziehen. Glättungsboarde sind üblicherweise rechteckige Tafeln z.B. aus Kunststoff. Vorzugsweise ist die Faser dabei mit der Keratin reduzierenden Zubereitung benetzt.

Eine weitere Möglichkeit der Haarglättung ist die Glättung mittels Glätteisen nach vorheriger Anwendung alkalischer Produkte. Solche alkalischen Umformungsmittel führen im Gegensatz zur Umformung mittels Keratin reduzierender und -oxidierender Zusammensetzungen nicht zu einer Umformung der Disulfidbrücken, sondern zu einer Zerstörung der Disulfidbrücken unter Bildung von Monosulfidbrücken. Je nach Konzentration und Anwendungsdauer der alkalischen Umformungsmittel werden auch Proteinketten hydrolytisch gespalten. Der pH-Wert der alkalischen Umformungsmittel liegt üblicherweise im Bereich von 11-14, vorzugsweise von 12-13.

Zur Erzielung permanenter, intensiver Färbungen mit guten Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln unter Kupplung mit einer oder mehreren Kupplerkomponenten die eigentlichen Farbstoffe aus. Die Oxidationsfärbemittel zeichnen sich durch intensive, hervorragende, lang anhaltende Färbeergebnisse aus.

Wünscht der Anwender zeitgleich sowohl eine permanente Umformung als auch eine Färbung seiner Haare, so kann er die zuvor beschriebenen Umformungs- und Färbe-Verfahren miteinander kombinieren. Entsprechende Verfahren zur gleichzeitigen Umformung und Färbung keratinischer Fasern wurden bereits in der Literatur beschrieben. So offenbart die DE 197 13 698 C1 ein Verfahren, bei welchem dem zur Fixierung des umgeformten Haares eingesetzten Oxidationsmittel ein Oxidationsfarbstoff-vorprodukt und/oder eine direktziehender Farbstoff zugesetzt wird.

WO 2012/038114 A2 beschreibt eine Mehrkomponenten-Verpackungseinheit zur Farbveränderung menschlicher Haare, welche zwei getrennt konfektionierte Zubereitungen enthält. Bei der ersten Zubereitung handelt es sich um ein Mittel zur Farbveränderung, dass mindestens ein Oxidationsfarbstoffvorprodukt enthalten kann. Die zweite Zubereitung ist ein Konditioniermittel enthaltend mindestens ein Reduktionsmittel.

Aus EP 352 375 A1, EP 1 287 812 A2 und DE 10 2005 061 023 A1 sind Verfahren zur gleichzeitigen Umformung und Färbung von Haaren bekannt, bei denen eine Keratin reduzierende Zubereitung eingesetzt wird, die bereits die notwendigen Farbstoffe und/oder Farbstoffvorprodukte enthält.

All diese aus dem Stand der Technik bekannten Verfahren basieren auf dem Einsatz eines Reduktionsmittels und einer nachfolgenden Fixierung durch ein Oxidationsmittel, wobei entweder der Reduktionsmittel-Komponente oder aber der nachfolgende angewendeten Oxidationsmittel-Komponente Farbstoffe (bzw. Farbstoffvorpodukte) zugesetzt werden.

Die während der Umformung stattfindende Reduktion der Disulid-Brücken im Keratinmaterial ist zwar die Voraussetzung für die Erzielung eines zufriedenstellenden Umformungs-Effektes, birgt jedoch gleichzeitig auch die Gefahr massiver Haarschädigungen. Denn die für die physikalische Stabilität des Haares wesentlichen, disulfidischen Cystin-Einheiten werden zunächst reduktiv zu Cystein gespalten, können jedoch bei der nachfolgenden Fixierung nicht vollständig wiederherstellt werden, da ein Teil des Cysteins sich in Nebenreaktionen zu Cysteinoxiden oder Cysteinsäure umwandelt. Das bei der Verformung in den plastischen Zustand überführte Haar erhärtet so zwar durch die oxidative Fixierung erneut, nimmt den ursprünglichen chemischen bzw. physikalischen Zustand jedoch nicht wieder ein.

Nach der Verformungs-Behandlung resultiert ein gelocktes - bzw. alternativ geglättetes - Haar, das innerlich unter Spannung steht, sich thermodynamisch nicht im stabilsten Zustand befindet, neue Aminosäuren (wie Cysteinoxide und Cysteinsäure) enthält und unvollständig quervernetzt ist. Der Anwender nimmt diesen Zustand als mehr oder weniger stark ausgeprägte Haarschädigung war, die - da sich der Einsatz der Reduktionsmittel prozessbedingt nicht vermeiden lässt - ein genereller Nachteil all dieser Umformungs-Verfahren ist.

Es war nun die Aufgabe der vorliegenden Erfindung, ein Umformungs- und Färbeverfahren für keratinhaltige Fasern, insbesondere für menschliches Haar, bereitzustellen, das ein sehr gutes und dauerhaftes Umformungs- und Färbeergebnis liefert, hierbei die Faser jedoch nicht schädigt und die Struktur der Faser schont.

Werden die zuvor beschriebenen Umformungs- und Färbeverfahren miteinander kombiniert oder sukzessive direkt hintereinander durchgeführt, so ergibt sich neben der Haarschädigung auch die Problematik der gegenseitigen Beeinflussung der beiden Verfahren, die zu ebenso unvorhersehbaren wie unerwünschten Farbergebnissen führen kann. So kann beispielsweise - wenn die Haare zunächst gefärbt und im Anschluss daran einer Dauerwelle bzw. reduktiven Glättung unterzogen werden - der nachfolgende Reduktionsvorgang beim zuvor gefärbten Haar zu einem Abbau der künstlichen Farbstoffe führen. Die Folge hiervon ist eine zu niedrige Farbintensität oder aber eine farbliche Abweichung von der gewünschten Nuance. Weiterhin reagiert auch unmittelbar zuvor gefärbtes Haar schneller auf die Dauerwellbehandlung, so dass der Umformungsgrad stärker als gewünscht ausfallen kann und es schlimmstenfalls zu einer Überkräuselung kommt. Andererseits ist auch bekannt, dass - wenn die Haare zunächst mit Dauerwell- bzw. Glättungsmitteln behandelt und danach gefärbt werden - das Haar durch die Dauerwell- bzw. Glättungsmittel aufnahmefähiger geworden ist und demzufolge die Farbstoffe schneller in die Haarfaser hinein penetrieren können. Die Folge hiervon ist ein zu dunkles Farbergebnis. Zudem kann auch die Dauerwelle bzw. Glättung in unerwünschter Weise durch die nachfolgende Färbung wieder abgeschwächt werden.

Ein Verfahren zur gleichzeitigen Umformung und Färben der Keratinfasern, welches diese Nachteile nicht besitzt, ist bislang aus dem Stand der Technik nicht bekannt. Eine weitere Aufgabe der vorliegenden Erfindung war es daher, Mittel zur gleichzeitigen Umformung und Färbung von keratinischen Fasern, insbesondere menschlichen Haaren, aufzufinden, welche sowohl die Umformung als auch die Färbung ermöglichen, jedoch ohne dass die beiden Verfahren sich gegenseitig in nachteiliger Weise beeinflussen, und ohne dass durch diese gegenseitige Beeinflussung ein unvorhersehbares Verformungs- und Färbeergebnis auftreten würde.

Aus der Literatur ist nun bekannt, dass Haare auch durch den Einsatz von Quellmitteln dauerhaft verformt werden können. Im Artikel J Soc Cosmet. Chem (1994), 45, 347 - 352, wird Recorcin eine glättende Wirkung zugesprochen - der Artikel befasst sich jedoch ausschließlich mit der Glättung von Haaren, eine Färbung wird in diesem Artikel an keiner Stelle thematisiert.

Im Zuge der zu dieser Erfindung führenden Arbeiten konnte nun überraschenderweise gefunden werden, dass eine gleichzeitige Umformung und oxidative Färbung der keratinischen Fasern möglich wird, wenn die keratinischen Fasern mit einem anwendungsbereiten Mittel behandelt werden, welches ein Oxidationsmittel, Oxidationsfarbstoffe vom Entwicklertyp und zusätzlich einen molaren Überschuss an Resorcin-Derivaten enthält.

Die im Überschuss vorhandenen Resorcin-Derivate sind hierbei sowohl für eine Glättung der Haare als auch - zusammen mit den weiteren Oxidationsfarbstoffvorprodukten und dem Oxidationsmittel - für eine Färbung der Haare verantwortlich. Auf diese Weise war es möglich, das Haar permanent umzuformen und gleichzeitig zu färben. Eine unvorhersehbare Nuancenverschiebung des Farbergebnisses oder eine unvorhersehbare Abschwächung des Verformungsergebnisses wurde in diesem Zusammenhang nicht beobachtet.

Darüber hinaus konnte mit Einsatz dieser Mittel auf zusätzliche Reduktionsmittel und auf starke Alkalisierungsmittel verzichtet werden, so dass die Faserstruktur weniger geschädigt und die Keratinfasern geschont wurden.

Zur Vermeidung von Inkompatibilitäten wird das anwendungsbereite Mittel, welches das Oxidationsmittel, die Oxidationsfarbstoffe vom Entwicklertyp und zusätzlich im molaren Überschuss bestimmte Resorcin-Derivate enthält, in Form einer Mehrkomponenten-Verpackungseinheit (Kit-of-parts) bereitgestellt, welche mindestens zwei getrennt voneinander konfektionierte Zubereitungen (A) und (B) umfasst, wobei die Oxidationsfarbstoffvorprodukte und Resorcine in der Zubereitung (A) enthalten sind und die Zubereitung (B) das Oxidationsmittel (Wasserstoffperoxid) enthält.

Es wird hier eine Mehrkomponenten-Verpackungseinheit (Kit-of-parts) beschrieben zum Umformen und Färben von keratinischen Fasern, insbesondere menschlichen Haaren, zur Anwendung in dem beanspruchten Verfahren, umfassend mindestens zwei getrennt voneinander konfektionierte Zubereitungen (A) und (B), wobei
- die erste Zubereitung (A) in einem kosmetischen Träger
   (A1) ein oder mehrere Resorcinderivate aus der Gruppe aus Resorcin, 2-Methylresorcin und 4-Chlorresorcin, und
   (A2) ein oder mehrere Oxidationsfarbstoffvorprodukte aus der Gruppe der p-Phenylendiamine, der p-Aminophenole, der 2,4,5,6-Tetraamiopyrimidine, der 4,5-Diaminopyrazole, und/oder deren physiologisch verträglichen Salzen enthält, und
- die zweite Zubereitung (B) in einem kosmetischen Träger
   (B1) Wasserstoffperoxid enthält,
dadurch gekennzeichnet, dass das molare Verhältnis aus allen in der Zubereitung (A) enthaltenen Resorcinderivaten (A1) zu allen in der Zubereitung (A) enthaltenen Oxidationsfarbstoffen vom Entwicklertyp (A2), d.h. das molare Verhältnis (A1)/(A2), bei einem Wert von mindestens 1,2 liegt.

Unter dem erfindungsgemäß verwendeten Begriff der "Umformung von keratinischen Fasern" wird eine permanente Umformung verstanden, die eine Welle oder Kräuselung der Keratinfasern oder auch eine Glättung der Keratinfasern sein kann. Permanente Umformungen sind Umformungen, die auch nach mindestens einer Haarwäsche noch vorhanden bzw. sichtbar sind.

Unter dem erfindungsgemäß verwendeten Begriff "Mittel zur Färbung von keratinischen Fasern" werden oxidative Färbemittel verstanden. Die Ausbildung der Färbung erfolgt durch Reaktion der Oxidationsfarbstoffvorprodukte vom Entwicklertyp (A2) mit den als Kuppler fungierenden Resorcin-Derivaten unter Einfluss des Oxidationsmittels Wasserstoffperoxid. Sind zusätzlich noch weitere Kuppler vorhanden, erfolgt die Färbung auch durch Reaktion der Oxidationsfarbstoffvorprodukte vom Entwicklertyp (A2) mit den zusätzlich enthaltenen Kupplern. Abhängig von der Menge des eingesetzten Oxidationsmittels wird die Keratinfaser während der Färbung gleichzeitig mehr oder weniger stark aufgehellt, da das Oxidationsmittel nicht nur den Farbstoffbildungsprozess von Entwicklern und Kupplern initiiert, sondern auch die haareigenen Pigmente (Melanine) oxidativ zerstört.

Unter keratinischen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose verwendet werden. Die Zubereitungen (A) und (B) enthalten alle wesentlichen Bestandteile jeweils in einem kosmetischen Träger. Bei dem kosmetischen Träger kann es sich um einen geeigneten wässrigen, oder wässrig-alkoholischen Träger handeln. Beispielsweise können das Mittel in Form einer Creme, einer Emulsion, eines Gels oder auch in Form einer tensidhaltigen schäumenden Lösung, wie beispielsweise eines Shampoos, eines Schaumaerosols, einer Schaumformulierung oder in Form einer anderen Zubereitung, die für die Anwendung auf dem Haar geeignet ist, auf die keratinischen Fasern aufgetragen werden.

Die Mehrkomponenten-Verpackungseinheit (Kit-of-parts) umfasst mindestens zwei getrennt voneinander konfektionierte Zubereitungen, eine erste Zubereitung (A) und eine zweite Zubereitung (B).

Die Zubereitung (A) enthält hierbei in einem kosmetischen Träger
- (A1) ein oder mehrere Resorcinderivate aus der Gruppe aus Resorcin, 2-Methylresorcin und 4-Chlorresorcin, und
- (A2) ein oder mehrere Oxidationsfarbstoffvorprodukte aus der Gruppe der p-Phenylendiamine, der p-Aminophenole, der 2,4,5,6-Tetraamiopyrimidine und der 4,5-Diaminopyrazole.

Bei den Resorcinderivaten (A1) handelt es sich um Resorcin, 2-Methylresorcin und/oder 4-Chlorresorcin. Eine gleichzeitige glättende und färbende Wirkung konnte bei Einsatz aller drei Resorcin-Derivate beobachtet werden.

Bei Resorcin handelt es sich um die Verbindung der Formel (I), bei 2-Methylresorcin handelt es sich um die Verbindung der Formel (II), und bei 4-Chlorresorcin handelt es sich um die Verbindung der Formel (III).

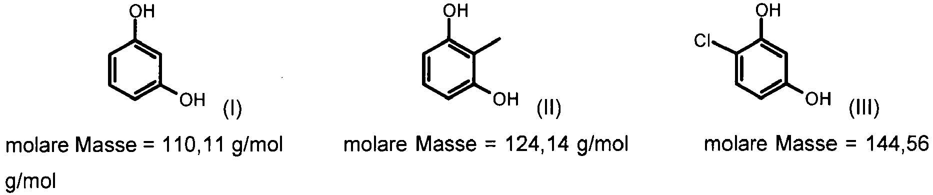

Damit sowohl eine optimale Umformung der Keratinfasern als auch ein intensives Farbergebnis möglich ist, werden die Resorcinderivate (A1) bevorzugt in bestimmten Mengenbereichen eingesetzt.

Besonders gute Ergebnisse wurden erhalten, wenn die erste Zubereitung (A) ein oder mehrere Resorcinderivate (A1) aus der Gruppe aus Resorcin, 2-Methylresorcin und 4-Chlorresorcin in einer Gesamtmenge von 1,0 bis 15,0 Gew-%, bevorzugt von 2,0 bis 13,0 Gew.-%, weiter bevorzugt von 3,0 bis 12,0 Gew.-%, noch weiter bevorzugt von 4,0 bis 11,0 Gew.-% und ganz besonders bevorzugt von 5,0 bis 10,5 Gew.-% enthält. Berechnungsgrundlage für diese Mengenangaben in Gew.-% ist hierbei das Gesamtgewicht aller Resorcinderivate aus der Gruppe (A1), das zum Gesamtgewicht der Zubereitung (A) in Relation gesetzt wird.

In einer ganz besonders bevorzugten Ausführungsform ist die Mehrkomponenten-Verpackungseinheit (Kit-of-parts) zur Anwendung in dem beanspruchten Verfahren dadurch gekennzeichnet, dass die erste Zubereitung (A) - bezogen auf das Gesamtgewicht der Zubereitung (A) - (A1) ein oder mehrere Resorcinderivate aus der Gruppe aus Resorcin, 2-Methylresorcin und 4-Chlorresorcin in einer Gesamtmenge von 1,0 bis 15,0 Gew-%, bevorzugt von 2,0 bis 13,0 Gew.-%, weiter bevorzugt von 3,0 bis 12,0 Gew.-%, noch weiter bevorzugt von 4,0 bis 11,0 Gew.-% und ganz besonders bevorzugt von 5,0 bis 10,5 Gew.-% enthält.

Bei den weiterhin in der Zubereitung (A) enthaltenen Oxidationsfarbstoffvorprodukten (A2) handelt es sich um Verbindungen aus der Gruppe der p-Phenylendiamine, der p-Aminophenole, der 2,4,5,6-Tetraamiopyrimidine und/oder der 4,5-Diaminopyrazole.

Bei Oxidationsfarbstoffvorprodukten aus der Gruppe der p-Phenylendiamine handelt es sich um Oxidationsfarbstoffvorprodukte, denen eine p-Phenylendiamin-Einheit zugrunde gelegt werden kann oder die Derivate hiervon sind. Dies bedeutet, dass die p-Phenylendiamin-Einheit der Oxidationsfarbstoffvorprodukte auch ein oder mehrfach substituiert vorliegen kann, wobei die Prämisse gilt, dass die Substitution dergestalt sein muss, dass noch eine oxidative Kupplungsreaktion mit den Resorcinen (bzw. den zusätzlich enthaltenen Kupplern) zum Farbstoff stattfinden kann.

Besonders geeignete Oxidationsfarbstoffvorprodukten aus der Gruppe der p-Phenylendiamine sind p-Phenylendiamin selbst, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan und/oder die physiologisch verträglichen Salzen dieser Verbindungen Ganz besonders geeignete Oxidationsfarbstoffvorprodukten aus der Gruppe der p-Phenylendiamine sind p-Toluylendiamin, 2-(2-Hydroxyethyl)-p-phenylendiamin, N,N-Bis-(2-hydroxyethyl)-p-phenylendiamin, 2-Methoxymethyl-p-phenylendiamin und/oder die physiologisch vertäglichen Salzen dieser Verbindungen.

Bei p-Toluylendiamin handelt es sich um eine Verbindung der Formel (IV) 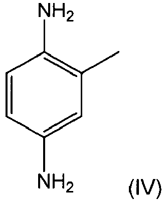 molare Masse = 122,17 g/mol

Bevorzugte physiologisch verträgliche Salze von p-Toluylendiamin sind insbesondere die Hydrochloride (Monohydrochlorid x HCl, oder Dihydrochlorid x 2 HCl), das Sulfat (x H₂SO₄) und die Hydrobromide (Monohydrobromid x HBr, oder Dihydrobromid x 2 HBr) der Verbindung. Ganz besonders bevorzugt ist p-Toluylendiamin Sulfat (Formel (IVa)). 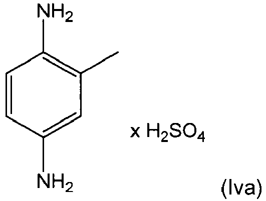 molare Masse = 220,25 g/mol

Bei 2-(2,5-Diaminophenyl)ethanol handelt es sich um die Verbindung der Formel (V). 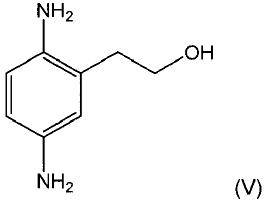 molare Masse = 152,20 g/mol

Bevorzugte physiologisch verträgliche Salze von 2-(2,5-Diaminophenyl)ethanol sind insbesondere die Hydrochloride (Monohydrochlorid x HCl, oder Dihydrochlorid x 2 HCl), das Sulfat (x H₂SO₄) und die Hydrobromide (Monohydrobromid x HBr, oder Dihydrobromid x 2 HBr) der Verbindung. Ganz besonders bevorzugt ist 2-(2,5-Diaminophenyl)ethanol Sulfat (Formel (Va)). 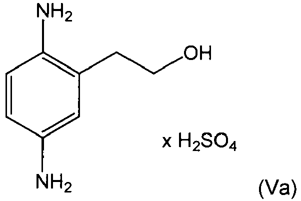 molare Masse = 250,27 g/mol

Bei N,N-Bis-(2-hydroxyethyl)-p-phenylendiamin handelt es sich um einer Verbindung der Formel (VI). 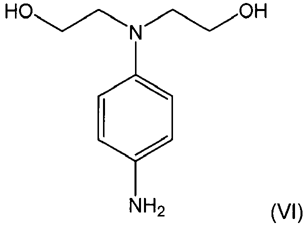 molare Masse = 196,25 g/mol

Bevorzugte physiologisch verträgliche Salze von N,N-Bis-(2-hydroxyethyl)-p-phenylendiamin sind insbesondere die Hydrochloride (Monohydrochlorid x HCl, oder Dihydrochlorid x 2 HCl), das Sulfat (x H₂SO₄) und die Hydrobromide (Monohydrobromid x HBr, oder Dihydrobromid x 2 HBr) der Verbindung. Ganz besonders bevorzugt ist N,N-Bis-(2-hydroxyethyl)-p-phenylendiamin Sulfat (Formel (VIa)). 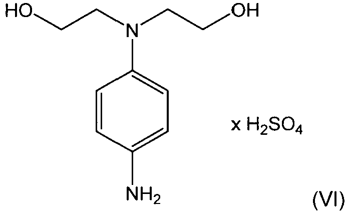 molare Masse = 294,32 g/mol

Bei 2-Methoxymethyl-p-phenylendiamin handelt es sich um einer Verbindung der Formel (VII) 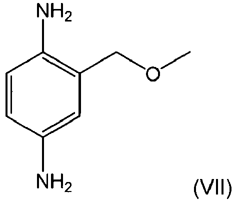 molare Masse = 152,20 g/mol

Bevorzugte physiologisch verträgliche Salze von 2-Methoxymethyl-p-phenylendiamin sind insbesondere die Hydrochloride (Monohydrochlorid x HCl, oder Dihydrochlorid x 2 HCl), das Sulfat (x H₂SO₄) und die Hydrobromide (Monohydrobromid x HBr, oder Dihydrobromid x 2 HBr) der Verbindung. Ganz besonders bevorzugt ist 2-Methoxymethyl-p-phenylendiamin Sulfat (Formel (VIIa)). 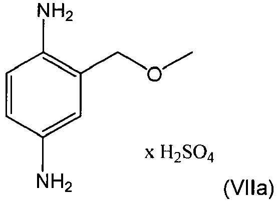 molare Masse = 250,27 g/mol

Bei Oxidationsfarbstoffvorprodukten aus der Gruppe der der p-Aminophenole handelt es sich um Oxidationsfarbstoffvorprodukte, denen eine p-Aminophenol-Einheit zugrunde gelegt werden kann oder die Derivate hiervon sind. Dies bedeutet, dass die p-Aminophenol-Einheit der Oxidationsfarbstoffvorprodukte auch ein oder mehrfach substituiert vorliegen kann, wobei die Prämisse gilt, dass die Substitution dergestalt sein muss, dass noch eine oxidative Kupplungsreaktion mit den Resorcinen (bzw. den zusatzlich enthaltenen Kupplern) zum Farbstoff stattfinden kann.

Besonders geeignete Oxidationsfarbstoffvorprodukte aus der Gruppe der p-Aminophenole sind p-Aminophenol selbst, 4-Amino-3-methylphenol, Bis-(2-hydroxy-5-aminophenyl)methan und/oder die physiologisch verträglichen Salze dieser Verbindungen.

Bevorzugte physiologisch verträgliche Salze von p-Aminophenol sind insbesondere das Hydrochloride (Monohydrochlorid x HCl), das Sulfat (x ½ H₂SO₄) und das Hydrobromid. Bevorzugte physiologisch verträgliche Salze von 4-Amino-3-methyphenol sind insbesondere das Hydrochloride (Monohydrochlorid x HCl), das Sulfat (x ½ H₂SO₄) und das Hydrobromid. Bevorzugte physiologisch verträgliche Salze von Bis-(2-hydroxy-5-aminophenyl)methan sind insbesondere das Hydrochloride (Monohydrochlorid x HCl), das Sulfat (x ½ H₂SO₄) und das Hydrobromid.

Bei Oxidationsfarbstoffvorprodukten aus der Gruppe der der 2,4,5,6-Tetraaminopyrimidine handelt es sich um Oxidationsfarbstoffvorprodukte, denen eine 2,4,5,6-Tetraamiopyrimidin-Einheit zugrunde gelegt werden kann oder die Derivate hiervon sind. Dies bedeutet, dass die 2,4,5,6-Tetraamiopyrimidin-Einheit der Oxidationsfarbstoffvorprodukte auch ein oder mehrfach substituiert vorliegen kann, wobei die Prämisse gilt, dass die Substitution dergestalt sein muss, dass noch eine oxidative Kupplungsreaktion mit den Resorcinen (bzw. den zusätzlich enthaltenen Kupplern) zum Farbstoff stattfinden kann.

Ein besonders geeignetes Oxidationsfarbstoffvorprodukten aus der Gruppe der 2,4,5,6-Tetraaminopyrimidine ist das 2,4,5,6-Tetraaminopyrimidin selbst oder seine physiologisch verträglichen Salze. Bei Oxidationsfarbstoffvorprodukten aus der Gruppe der der 4,5-Diaminopyrazole handelt es sich um Oxidationsfarbstoffvorprodukte, denen eine 4,5-Diaminopyrazol-Einheit zugrunde gelegt werden kann oder die Derivate hiervon sind. Dies bedeutet, dass die 4,5-Diaminopyrazol-Einheit der Oxidationsfarbstoffvorprodukte auch ein oder mehrfach substituiert vorliegen kann, wobei die Prämisse gilt, dass die Substitution dergestalt sein muss, dass noch eine oxidative Kupplungsreaktion mit den Resorcinen (bzw. den zusätzlich enthaltenen Kupplern) zum Farbstoff stattfinden kann.

Ein besonders geeignetes Oxidationsfarbstoffvorprodukten aus der Gruppe der4,5-Diaminopyrazole ist 4,5-Diamino-1-(2-hydroxyethyl)pyrazol selbst oder seine physiologisch verträglichen Salze. Bei 4,5-Diamino-1-(2-hydroxyethyl)pyrazol handelt es sich um einer Verbindung der Formel (VIII) 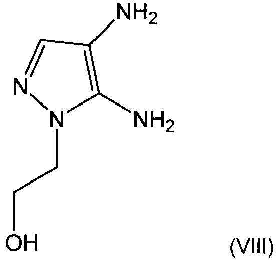 molare Masse = 142,16 g/mol

Bevorzugte physiologisch verträgliche Salze von 4,5-Diamino-1-(2-hydroxyethyl)-1H-pyrazol sind insbesondere die Hydrochloride (Monohydrochlorid x HCl, oder Dihydrochlorid x 2 HCl), das Sulfat (x H₂SO₄) und die Hydrobromide (Monohydrobromid x HBr, oder Dihydrobromid x 2 HBr) der Verbindung. Ganz besonders bevorzugt ist 4,5-Diamino-1-(2-hydroxyethyl)-1H-pyrazol Sulfat (Formel (VIIIa)). 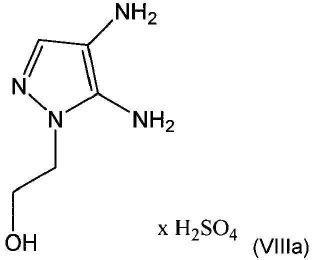 molare Masse = 240,23 g/mol

Die besten Farbergebnisse konnten erhalten werden, wenn die Resorcine (A1) mit ganz bestimmten Oxidationsfarbstoffen vom Entwicklertyp (A2) zur Reaktion gebracht wurden. Besonders geeignete Oxidationsfarbstoffe vom Entwicklertyp (A2) sind ausgewählt aus der Gruppe aus p-Phenylendiamin, p-Toluylendiamin, 2-(2-Hydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)methan, p-Aminophenol, 4-Amino-3-methylphenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin und/oder deren physiologisch verträglichen Salzen.

Da die Zubereitungen (A) und (B) mit dem Haar - und damit auch mit der Kopfhaut - der Anwender in Kontakt kommen, müssen die Salze der Oxidationsfarbstoffe vom Entwicklertyp (A2) physiologisch verträglich sein. Physiologisch verträglich bedeutet in diesem Zusammenhang zum Einsatz im kosmetischen Mittel (d.h. zur Anwendung auf dem menschlichen Haar und der menschlichen Haut) geeignet. Besonders bevorzugte physiologisch verträgliche Salze sind die Hydrochloride, die Hydrobromide, die Hemisulfate, die Sulfate, die p-Toluolsulfonsate, die Benzolsulfonate, die Acetate, die Lactate und/oder die Tartrate der Oxidationsfarbstoffe vom Entwicklertyp (A2). Ganz besonders bevorzugt sind deren Hydrochloride (Monohydrochlorid x HCl oder Dihydrochloride x 2 HCl), Sulfate (x ½ H₂SO₄) und Hydrobromide (Monohydrobormide x HBr oder Dihydrobromide x 2 HBr).

In einer weiteren ganz besonders bevorzugten Ausführungsform ist eine erfindungsgemäße Mehrkomponenten-Verpackungseinheit (Kit-of-parts) dadurch gekennzeichnet, dass die erste Zubereitung (A) einen oder mehrere Oxidationsfarbstoffvorprodukte vom Entwicklertyp (A2) enthält, die ausgewählt sind aus der Gruppe aus p-Phenylendiamin, p-Toluylendiamin, 2-(2-Hydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)methan, p-Aminophenol, 4-Amino-3-methylphenol, 4,5-Diamino-1-(2-hydroxyethyl)-pyrazol, 2,4,5,6-Tetraaminopyrimidin, und/oder deren physiologisch verträglichen Salzen. Bevorzugt sind das oder die Oxidationsfarbstoffvorprodukte vom Entwicklertyp (A2) in bestimmten Mengenbereichen in der Zubereitung (A) enthalten. Besonders intensive Färbungen werden erhalten, wenn die erste Zubereitung (A) einen oder mehrere Oxidationsfarbstoffvorprodukte vom Entwicklertyp (A2) in einer Gesamtmenge von 0,1 bis 2,5 Gew.-%, bevorzugt von 0,2 bis 2,3 Gew.-%, weiter bevorzugt von 0,6 bis 1,7 Gew.-% und besonders bevorzugt von 0,9 bis 1,1 Gew.-% enthält. Berechnungsgrundlage für diese Mengenangaben in Gew.-% ist hierbei das Gesamtgewicht aller Oxidationsfarbstoffvorprodukte vom Entwicklertyp aus der Gruppe (A2), das zum Gesamtgewicht der Zubereitung (A) in Relation gesetzt wird.

In einer weiteren ganz besonders bevorzugten Ausführungsform ist die Mehrkomponenten-Verpackungseinheit (Kit-of-parts) zur Anwendung in dem beanspruchten Verfahren dadurch gekennzeichnet, dass die erste Zubereitung (A) - bezogen auf das Gesamtgewicht der Zubereitung (A) - einen oder mehrere Oxidationsfarbstoffvorprodukte vom Entwicklertyp (A2) in einer Gesamtmenge von 0,1 bis 2,5 Gew.-%, bevorzugt von 0,2 bis 2,3 Gew.-%, weiter bevorzugt von 0,6 bis 1,7 Gew.-% und besonders bevorzugt von 0,9 bis 1,1 Gew.-% enthält.

Ein ganz zentrales und erfindungswesentliches Merkmal der vorliegenden Erfindung besteht darin, dass in der Zubereitung (A) das molare Verhältnis aus allen Resorcinderivaten (A1) zu allen Oxidationsfarbstoffvorprodukten vom Entwicklertyp (A2), d.h. das molare Verhältnis (A1)/(A2), bei einem Wert von mindestens 1,2 liegt. Mit anderen Worten werden die Resorcinderivate (A1) im Vergleich zu den Oxidationsfarbstoffvorprodukten vom Entwicklertyp (A2) in einem mindestens 1,2-fachen molaren Überschuss eingesetzt.

Durch die im molaren Überschuss enthaltenen Resorcinderivate (A1) wird gewährleistet, dass neben der Färbung der Keratinfasern, die durch Reaktion der Resorcine (A1) mit den Oxidationsfarbstoffvorprodukten vom Entwicklertyp (A2) unter Ausbildung von Farbstoffen zustande kommt, auch noch eine Glättung erzielt werden kann. Ohne auf diese Theorie beschränkt zu sein, wird vermutet, dass die Glättung durch die überschüssigen Resorcine (A1) hervorgerufen wird, da die Resorcine (A1), die bei der Farbstoffbildungsreaktion nicht verbraucht werden, als Quellmittel fungieren können, wodurch sich die Form der Keratinfasern permanent beeinflussen lässt.

Ohne dass durch den molaren Überschuss der Resorcine (A1) die Farbnuance nachteilig beeinflusst würde, wird auf diese Weise zusätzlich eine permanente Lockung oder Glättung der Keratinfasern ermöglicht.

Die Molmasse (Einheit g/mol) einer Verbindung ist definiert als Masse (Einheit g) pro Stoffmenge (Einheit Mol).

Unter der Stoffmenge wird die Quantität einer Stoffportion auf der Grundlage der Anzahl der jeweils darin enthalten Teilchen verstanden. Die Einheit der Stoffmenge ist die Basiseinheit Mol (mol bzw. mmol).

Das quantitative Verhältnis der Resorcine (A1) zu den Oxidationsfarbstoffvorprodukten vom Entwicklertyp (A2) wird als molares Verhältnis definiert, da die Oxidationsfarbstoffvorprodukte vom Entwicklertyp (A2) mit einer bestimmten Anzahl von Resorcinen-Molekülen (A1) reagieren.

Da die Zubereitung (B) keine Oxidationsfarbstoffvorprodukte (d.h. weder Verbindungen aus der Gruppe (A1) noch Verbindungen aus der Gruppe (A2)) enthält, entspricht das molare Verhältnis (A1)/(A2) in der Zubereitung (A) auch dem molaren Verhältnis (A1)/(A2) in dem anwendungsbereiten Umformungs- bzw. Färbemittel, das durch Vermischen der Zubereitungen (A) und (B) hergestellt wird.

Die Molmenge der in der Zubereitung (A) enthaltenen Verbindungen (A1) und (A2) kann jeweils durch
die Division der Einsatzmengen durch die entsprechenden molaren Mengen erhalten werden.

### Beispiel:

100 g einer Zubereitung (A) enthalten
   (A1) 5,00 g Resorcin (molare Masse = 110,11 g/mol) und 3,00 g 2-Methylresorcin (molare Masse = 124,14 g/mol)
Damit enthält die Zubereitung (A)
   (A1) 45,4 mmol Resorcin und 24,2 mmol 2-Methylsesorcin
Summe = 69,6 mmol Resorcine (A1)
Weiterhin enthält die Zubereitung (A)
   (A2) 1,50 g p-Toluylendiamin Sulfat (molare Masse = 220,25 g/mol) und 0,50 g 4,5-Diamino-1-(2-hydroxyethyl)-1 H-pyrazol Sulfat (molare Masse = 240,23 g/mol)
Damit enthält die Zubereitung (A)
   (A2) 6,81 mmol p-Toluylendiamin Sulfat und 2,08 mmol 4,5-Diamino-1-(2-hydroxyethyl)-1H-pyrazol Sulfat
Summe = 8,89 mmol Oxidationsfarbstoffvorprodukte der Gruppe (A2)
Das molare Verhältnis (A1)/(A2) in Zubereitung (A) liegt bei 69,6 mmol/8,89 mmol = 7,83
Das molare Verhältnis (A1)/(A2) im anwendungsbereiten Mittel (welches eine Mischung der Zubereitungen (A) und (B) ist) liegt ebenfalls bei 7,83.

Damit die Resorcinderivate (A1) sowohl eine Umformung als auch eine Färbung der Keratinfasern hervorrufen können, müssen sie in der Zubereitung (A) im Vergleich zu den Oxidationsfarbstoffen vom Entwicklertyp (A2) in einem mindestens 1,2-fachen molaren Überschuss enthalten sein. Bereits ab einem 1,2-fachen molaren Überschuss ist neben dem Färbe-Effekt auch ein Umformungs-Effekt zu beobachten. Der Umformungs-Effekt wird jedoch noch ausgeprägter, wenn der molare Überschuss der Resorcine, d.h. das Verhältnis (A1)/(A2), in der Zubereitung (A) noch weiter erhöht wird. Überraschenderweise wird das Farbergebnis durch die Erhöhung der Menge der in der Zubereitung (A) enthaltenen Resorcine aus der Gruppe (A1) nicht nachteilig beeinflusst.

Eine ganz besonders gute Färbung und Umformung konnte beobachtet werden, wenn das molare Verhältnis aus allen im Mittel enthaltenen Resorcinderivaten (A1) zu allen im Mittel enthaltenen Oxidationsfarbstoffen vom Entwicklertyp (A2), d.h. das molare Verhältnis (A1)/(A2), bei einem Wert von 1,3 bis 60,0, bevorzugt von 1,5 bis 50,0, bevorzugt von 2,0 bis 45,0, weiter bevorzugt von 4,0 bis 35,0 und besonders bevorzugt von 7,0 bis 32,0 lag.

In einer weiteren explizit ganz besonders bevorzugten Ausführungsform ist die Mehrkomponenten-Verpackungseinheit (Kit-of-parts) zur Anwendung in dem beanspruchten Verfahren daher dadurch gekennzeichnet, dass das molare Verhältnis aus allen in der Zubereitung (A) enthaltenen Resorcinderivaten (A1) zu allen in der Zubereitung (A) enthaltenen Oxidationsfarbstoffvorprodukten vom Entwicklertyp (A2), d.h. das molare Verhältnis (A1)/(A2), bei einem Wert von 1,3 bis 60,0, bevorzugt von 1,5 bis 50,0, weiter bevorzugt von 2,0 bis 45,0, noch weiter bevorzugt von 4,0 bis 35,0 und besonders bevorzugt von 7,0 bis 32,0 liegt.

Neben den Oxidationsfarbstoffvorprodukten aus den vorbeschriebenen Gruppen (A1) und (A2) kann die Zubereitung (A) zusätzlich auch noch einen oder weitere Kuppler enthalten, die von den strukturell von den Resorcinderivaten (A1) verschieden sind. Zusätzliche Kuppler können eingesetzt werden, um den gewünschten Farbton zu modifizieren.

Kupplerkomponenten bilden im Rahmen der oxidativen Färbung allein keine signifikante Färbung aus, sondern benötigen stets die Gegenwart von Entwicklerkomponenten. Kupplerkomponenten erlauben mindestens eine Substitution eines chemischen Restes des Kupplers durch die oxidierte Form der Entwicklerkomponente. Dabei bilden sich kovalente Bindungen zwischen Kuppler- und Entwicklerkomponente aus.

Als erfindungsgemäß geeignete Kupplerkomponente wird bevorzugt mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate, wie beispielsweise 6-Hydroxybenzomorpholin oder 6-Aminobenzomorpholin,
- Chinoxalinderivate, wie beispielsweise 6-Methyl-1,2,3,4-tetrahydrochinoxalin.

Gemische aus zwei oder mehreren Verbindungen aus einer oder mehreren dieser Klassen sind im Rahmen dieser Ausführungsform ebenso erfindungsgemäß.

Die zusätzlichen Kuppler bilden in Kombination mit den Oxidationsfarbstoffvorprodukten aus der Gruppe (A2) sehr intensive Färbungen aus. Bevorzugt sind zusätzlich einzusetzende Kuppler, die ausgewählt sind aus der Gruppe aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxy-ethylamino)-1-methylbenzol, 2-({3-[(2-Hydroxyethyl)amino]-4-methoxy-5-methylphenyl}amino)-ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin und/oder 7-Hydroxyindolin und deren physiologisch verträglichen Salzen.

In einer weiteren Ausführungsform ist die Mehrkomponenten-Verpackungseinheit (Kit-of-Parts) zur Anwendung in dem beanspruchten Verfahren dadurch gekennzeichnet, dass die Zubereitung (A) zusätzlich mindestens ein oder mehrere Kuppler aus der Gruppe enthält, die gebildet wird aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-di-aminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin und/oder deren physiologisch verträglichen Salzen.

Die Zubereitung (A) kann prinzipiell zusatzlich eine oder mehrere Kupplerkomponenten aus der vorgenannten Gruppe in einer Gesamtmenge von 0,001 bis 3,0 Gew.-%, bevorzugt von 0,025 bis 1,5 Gew.-%, weiter bevorzugt von 0,05 bis 1,0 Gew.-% und besonders bevorzugt von 0,1 bis 0,5 Gew.-% - bezogen auf das Gesamtgewicht der Zubereitung (A) - enthalten.

Zur weiteren Nuancenentwicklung kann die Zubereitung (A) zusätzlich auch mindestens einen direktziehenden Farbstoff aus der Gruppe der anionischen, nichtionischen und/oder kationischen Farbstoffe enthalten.

Besonders bevorzugt handelt es sich hierbei um einen oder mehrere nichtionische direktziehende Farbstoffe aus der Gruppe, HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Geeignete kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls geeignete kationische direktziehende Farbstoffe.

Als geeignete anionische Farbstoffe oder Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, lodeosin), Acid Red 52 (CI 45100, Food Red 106, Solar Rhodamine B, Acid Rhodamine B, Red n° 106 Pontacyl Brilliant Pink), Acid Red 73 (CI 27290), Acid Red 87 (Eosin, CI 45380), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, Cl 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Neben der Zubereitung (A) umfasst die Mehrkomponenten-Verpackungseinheit (Kit-of-parts) zur Anwendung in dem beanspruchten Verfahren weiterhin die getrennt von der Zubereitung (A) konfektionierte Zubereitung (B). Die Zubereitung (B) enthält in einem kosmetischen Träger (B1) Wasserstoffperoxid. Durch Reaktion des Wasserstoffperoxids mit den Oxidationsfarbstoffvorprodukten bilden sich in einem oxidativen Färbeprozess die Farbstoffe aus. Zur Verhinderung einer vorzeitigen Reaktion können die Verbindungen (A1) und (A2) nicht zusammen mit dem Wasserstoffperoxid gelagert werden.

In einer bevorzugten Ausführungsform wird Wasserstoffperoxid selbst als wässrige Lösung in der Zubereitung (B) eingesetzt. Die Konzentration einer Wasserstoffperoxid-Lösung in der Zubereitung (B) wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte Zubereitungen (B) sind dadurch gekennzeichnet, dass sie 0,1 bis 6,0 Gew.-%, bevorzugt von 0,15 bis 5,0 Gew.-%, weiter bevorzugt von 0,2 bis 4,5 Gew.-% und ganz besonders bevorzugt von 0,3 bis 4,2 Gew.-% Wasserstoffperoxid, jeweils bezogen auf das Gesamtgewicht der Zubereitung (B), enthalten.

In einer weiteren explizit ganz besonders bevorzugten Ausführungsform ist die Mehrkomponenten-Verpackungseinheit (Kit-of-parts) zur Anwendung in dem beanspruchten Verfahren daher dadurch gekennzeichnet, dass die zweite Zubereitung (B) - bezogen auf das Gesamtgewicht der Zubereitung (B) - Wasserstoffperoxid in einer Menge von 0,1 bis 12,0 Gew.-%, bevorzugt von 0,15 bis 5,0 Gew.-%, weiter bevorzugt von 0,2 bis 4,5 Gew.-% und ganz besonders bevorzugt von 0,3 bis 4,2 Gew.-% enthält.

Durch Applizierung des anwendungsbereiten Umformungs- und Färbemittels, d.h. durch Anwendung das Gemisches aus den Zubereitungen (A) und (B), können keratinische Fasern sowohl oxidativ gefärbt als auch permanent verformt werden. Auf die Anwendung von weiteren Reduktionsmitteln, wie sie für die aus dem Stand der Technik bekannten Wellmittel oder Glättungsmittel obligatorisch sind, kann aus diesem Grund verzichtet werden. Dieser Verzicht auf die Reduktionsmittel macht eine Umformung und Färbung bei größtmöglicher Schonung der Keratinfaser möglich. Haarschädigungen könnten daher bei Anwendung der erfindungsgemäßen Mehrkomponenten-Verpackungseinheit bestmöglich vermieden werden.

Aus diesem Grund sind auch die Mehrkomponenten-Verpackungseinheiten (Kit-of-parts) ganz besonders bevorzugt, die dadurch gekennzeichnet sind, dass die Zubereitung (A) frei von Reduktionsmitteln ist. Die Zubereitung (B) enthält als Oxidationsmittel Wasserstoffperoxid, so dass der Zusatz von Reduktionsmitteln zur Zubereitung (B) unerwünschte Reaktionen zwischen Oxidationsmittel und Reduktionsmittel hervorrufen würde und demnach ebenfalls nicht angezeigt ist. Unter einer Zubereitung (A), die frei von Reduktionsmitteln ist, wird eine Zubereitung verstanden, deren Gesamtgehalt an Reduktionsmitteln aus der Gruppe aus Thioglykolsäure, Thiomilchsäure, Cystein und den Salzen dieser Verbindungen - bezogen auf das Gesamtgewicht der Zubereitung (A) - unterhalb von 1,0 Gew.-%, bevorzugt unterhalb von 0,5 Gew.-%, weiter bevorzugt unterhalb von 0,1 Gew.-% und besonders bevorzugt unterhalb von 0,05 Gew.-% liegt.

In einer weiteren ganz besonders bevorzugten Ausführungsform ist die Mehrkomponenten-Verpackungseinheit (Kit-of-parts) zur Anwendung in dem beanspruchten Verfahren dadurch gekennzeichnet, dass die erste Zubereitung (A) - bezogen auf das Gesamtgewicht der Zubereitung (A) - reduzierende Substanzen aus der Gruppe aus Thioglykolsäure, Thiomilchsäure, Cystein und den Salzen dieser Verbindungen in einer Gesamtmenge von weniger als 1,0 Gew.-%, bevorzugt von weniger als 0,5 Gew.-%, weiter bevorzugt von weniger als 0,1 Gew.-% und besonders bevorzugt von weniger als 0,05 Gew.-% enthält.

Zur Verhinderung der Autoxidation der Oxidationsfarbstoffvorprodukte (A2) kann die Zubereitung (A) geringe Mengen an Reduktionsmitteln aus der Gruppe der Sulfite (z.B. Natriumsulfit, Natirumbisulfit, Ammoniumsulfit, Kaliumsulfit usw.) oder Ascorbinsäure enthalten. Die Einsatzmengen dieser Reduktionsmittel liegen hierbei unterhalb von 1,0 Gew.-%, bevorzugt unterhalb von 0,5 Gew.-%, noch weiter bevorzugt unterhalb von 0,1 Gew.-% (jeweils berechnet als das Gesamtgewicht der Reduktionsmittel aus der Gruppe der Sulfite und Ascorbinsäure bezogen auf das Gesamtgewicht der Zubereitung (A)). Diese Reduktionsmittel wirken jedoch - insbesondere in diesen geringen Einsatzmengen - auf die keratinischen Fasern nicht umformend, sondern sollen lediglich eine durch die Abreaktion der Oxidationsfarbstoffvorprodukte (A2) mit Luftsauerstoff hervorgerufene unattraktive Verfärbung der Zubereitung (A) verhindern. Um die Haarschädigung komplett zu minimieren, dann es von Vorteil sein, auf diese Reduktionsmittel ebenfalls zu verzichten.

In einer weiteren ganz besonders bevorzugten Ausführungsform ist die Mehrkomponenten-Verpackungseinheit (Kit-of-parts) zur Anwendung in dem beanspruchten Verfahren dadurch gekennzeichnet, dass die erste Zubereitung (A) - bezogen auf das Gesamtgewicht der Zubereitung (A) - reduzierende Substanzen aus der Gruppe aus Thioglykolsäure, Thiomilchsäure, Cystein und den Salzen dieser Verbindungen, Natriumsulfit, Natriumbisulfit, Ammoniumsulfit, Ammoniumbisulfit, Kaliumsulfit und Kaliumbisulfit in einer Gesamtmenge von weniger als 1,0 Gew.-%, bevorzugt von weniger als 0,5 Gew.-%, weiter bevorzugt von weniger als 0,1 Gew.-% und besonders bevorzugt von weniger als 0,05 Gew.-% enthält.

Zur weiteren Verbesserung des Umformungs- und Färbeergebnisses hat es sich als vorteilhaft erwiesen, wenn die Zubereitung (A) und/oder die Zubereitung (B) mindestens ein weiteres Quellmittel enthält. Als Quellmittel wird vorzugsweise eine Verbindung aus der Gruppe aus Harnstoff und Harnstoffderivaten, Guanidin und dessen Derivaten, Arginin und dessen Derivaten, Wasserglas, Imidazol und dessen Derivaten, Histidin und dessen Derivaten, Benzylalkohol, Glycerin, Glykol und Glykolether, Propylenglykol und Propylenglykolether, beispielsweise Propylenglykolmonoethylether, Carbonaten, Hydrogencarbonaten, Diolen und Triolen, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol aber auch Sorbit oder Pyrrolidoncarbonsäure eingesetzt. Eine besonders vorteilhafte Haarglättung und besonders vorteilhafte Färbeergebnisse werden durch den Einsatz eines Quellmittels aus der Gruppe aus Harnstoff (Urea), Glycerin (Propan-1,2,3-triol), Sorbit (D-Glucit) und/oder Pyrrolidoncarbonsäure (5-Oxopyrrolidin-2-carbonsäure), insbesondere Harnstoff, erzielt. Der Einsatz dieser spezifischen Quellmittel ist daher erfindungsgemäß bevorzugt.

Der Gewichtsanteil des Quellmittels am Gesamtgewicht der Zubereitung (A) bzw. der Zubereitung (B) beträgt vorzugsweise 0,2 bis 15 Gew.-%, bevorzugt 1,0 bis 12 Gew.-% und insbesondere 2,0 bis 10 Gew.-%.

In einer weiteren besonders bevorzugten Ausführungsform ist die Mehrkomponenten-Verpackungseinheit (Kit-of-parts) zur Anwendung in dem beanspruchten Verfahren daher dadurch gekennzeichnet, dass die Zubereitung (A) und/oder die Zubereitung (B) mindestens ein Quellmittel, bevorzugt aus der Gruppe aus Harnstoff (Urea), Glycerin (Propan-1,2,3-triol), Sorbit (D-Glucit) und/oder Pyrrolidoncarbonsäure (5-Oxopyrrolidin-2-carbonsäure) enthält.

Die Zubereitungen (A) und/oder (B) können zur Verstärkung der aufhellenden Wirkung weitere Bleichkraftverstärker enthalten, wie beispielsweise Tetraacetylethylendiamin (TAED), 1,5-Diacetyl-2,4-dioxohexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat, sowie N-Methyl-3,4-dihydroisochinolinium-p-toluolsulfonat.

Zur weiteren Steigerung der Aufhellung können die Zubereitungen (A) und/oder (B) zusätzlich auch noch mindestens eine SiO₂-Verbindung, wie Kieselsäure oder Silicate, insbesondere Wassergläser, zugesetzt sein. Die SiO2-Verbindung kann hierbei in der Färbezubereitung (A) und/oder in der Oxidationsmittelzubereitung (B) enthalten sein. Es kann erfindungsgemäß bevorzugt sein, die SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf das Gesamtgewicht der Färbezubereitung (A) bzw. auf das Gesamtgewicht der Oxidationsmittelzubereitung (B), einzusetzen. Die Mengenangaben geben dabei jeweils den Gehalt der SiO₂-Verbindungen (ohne deren Wasseranteil) in den Mitteln wieder.

Die Zubereitungen (A) und/oder (B) können weiterhin zusätzliche Wirk-, Hilfs- und Zusatzstoffe enthalten, um die Färbe- bzw. Aufhellleistung zu verbessern und weitere gewünschte Eigenschaften der Mittel einzustellen.

Vorzugsweise werden die anwendungsbereiten Färbemittel als flüssige Zubereitung bereitgestellt und den Mitteln daher gegebenenfalls zusätzlich eine weitere oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, zwitterionischen, amphoteren und nichtionischen Tensiden und Emulgatoren ausgewählt.

Erfindungsgemäß geeignete Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül.

Erfindungsgemäß geeignete Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß geeignete Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Die anwendungsbereiten Farbveränderungsmittel können auch mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind anionische, synthetische Polymere, kationische, synthetische Polymere, natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen, nichtionische, vollsynthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazoliniummethochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, jeweils bezogen auf das Gesamtgewicht der Färbezubereitung (A) und/oder der Oxidationsmittelzubereitung (B), eingesetzt.

Die Färbung und Umformung der keratinischen Fasern kann bei pH-Werten im Bereich von 2 bis 12, erfolgen, jedoch ist eine Färbung und Umformung im alkalischen Bereich bevorzugt. Wesentlich für das Färbe- und Umformungsergebnis ist hierbei der pH-Wert des anwendungsbereiten Mittels, d.h. der Mischung der Zubereitungen (A) und (B). Aus Stabilitätsgründen wird die Zubereitung (B), welche das Oxidationsmittel Wasserstoffperoxid enthält, auf einen sauren pH-Wert von 1,0 bis 6,0, bevorzugt von 2,0 bis 5,0 eingestellt. Die Zubereitung (A) wird bevorzugt auf einen alkalischen pH-Wert von 7,0 bis 11,5, bevorzugt von 8,0 bis 10,5 eingestellt. Der pH-Wert der Mischung aus den Zubereitungen (A) und (B) liegt bevorzugt ebenfalls im alkalischen Bereich von 7,5 bis 10,5.

Zur Einstellung der sauren pH-Werte kommen prinzipiell alle Verbindungen in Frage, die zur Abgabe eines Protons (einwertige Säure) oder mehrerer Protonen (mehrwertige Säure) in der Lage sind. Als anorganische Säuren können beispielsweise Mineralsäuren wie Salzsäure, Schwefelsäure und Phosphorsäure, bevorzugt in ihrer mit Wasser verdünnten Form, verwendet werden. Auch organische Säuren können in den erfindungsgemäßen Formulierungen eingesetzt werden. Typische Vertreter für organische Säuren sind aliphatische Mono- und Dicarbonsäuren wie beispielsweise Essigsäure, Propionsäure, Oxalsäure und 1,3-Propandisäure sowie aromatische Carbonsäuren wie beispielsweise Benzoesäure. Weitere erfindungsgemäße organische Säuren sind Hydroxycarbonsäuren wie Glykolsäure, Zitronensäure, Weinsäure, Äpfelsäure und Milchsäure. Auch ungesättigte Mono- oder Dicarbonsäuren wie beispielsweise Fumarsäure oder α-Ketocarbonsäuren wie beispielsweise Brenztraubensäure (2-Oxopropionsäure) sind erfindungsgemäß.

Aufgrund der für kosmetische Mittel bestehenden formulierungstechnischen und gesetzlichen Anforderungen sind jedoch geruchsarme, für den Einsatz in Kosmetika zugelassene Säuren am besten geeignet zur Entwicklung von Haarbehandlungsmitteln mit guter Färbeleistung. Haarfärbemittel, die mindestens eine Säure ausgewählt aus Zitronensäure, Weinsäure, Äpfelsäure, Milchsäure, 1-Hydroxyethan-1,1-diphosphonsäure, 2,6-Dipicolinsäure und Benzoesäure enthalten, sind daher bevorzugt.

Die zur Einstellung der bevorzugten pH-Werte - insbesondere des pH-Wertes der Zubereitung (A) - erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Bei den im Sinne dieser Erfindung gemessenen pH-Werten handelt es sich um pH-Werte, die bei einer Temperatur von 22 °C gemessen wurden. Zur Messung des pH-Wertes eignen sich insbesondere Glaselektroden, die beispielsweise in Form einer Einstabmesskette ausgeführt sein können.

Die erfindungswesentlichen Zubereitungen der Mehrkomponenten-Verpackungseinheit (Kit-of-parts) sind die Zubereitungen (A) und (B), da deren Vermischung und Applizierung für das Färbe- und Umformungs-Ergebnis der Keratinfasern verantwortlich ist. Zur Erzielung weiterer Effekte kann die Mehrkomponenten-Verpackungseinheit jedoch auch noch weitere Zubereitungen umfassen, wie beispielsweise eine dritte Conditioner Zubereitung (C), die pflegende Inhaltsstoffe wie kationische Polymere oder kationische Tenside enthalten kann.

Zur Vereinfachung des Anwendungsprozesses und zur Verkürzung des Anwendungszeitraums ist es jedoch bevorzugt, wenn die Mehrkomponenten-Verpackungseinheit genau diese beiden Zubereitungen (A) und (B) umfasst und keine dritte Zubereitung enthält, die als Vor- oder Nachbehandlungsmittel, auf die keratinischen Fasern zu applizieren wäre.

In einer weiteren besonders bevorzugten Ausführungsform ist die Mehrkomponenten-Verpackungseinheit (Kit-of-parts) zur Anwendung in dem beanspruchten Verfahren dadurch gekennzeichnet, dass sie zwei getrennt voneinander konfektionierte Zubereitungen (A) und (B) umfasst.

Hiermit ist gemeint, dass die Mehrkomponenten-Verpackungseinheit genau die beiden getrennt voneinander konfektionierte Zubereitungen (A) und (B) und keine weitere Zubereitung (C) umfasst. Die erfindungsgemäßen Mehrkomponenten-Verpackungseinheiten können in Verfahren zum gleichzeitigen Umformen und Färben von keratinischen Fasern, insbesondere menschlichen Haaren, eingesetzt werden.

Das erfindungsgemäße Verfahren zum Umformen und Färben von menschlichen Haaren, umfasst die folgenden Schritte:
(I) Herstellung eines anwendungsbereiten Umformungs- und Färbemittels durch Vermischen einer Zubereitung (A) mit einer Zubereitung (B), wobei die Zubereitungen (A) und (B) bei der Beschreibung des ersten Erfindungsgegenstands im Detail offenbart sind,
(II) Applikation des in Schritt (I) hergestellten anwendungsbereiten Umformungs- und Färbemittels auf die Haare,
(III) mechanische Umformung der Haare,
(IV) Spülen der Haare,
(V) gegebenenfalls trocknen der Haare.

Zur Herstellung des anwendungsbereiten Umformungs- und Färbemittels können die Zubereitungen (A) und (B) in einem Gewichtsverhältnis von 1:10 bis 10:1 miteinander vermischt werden. Besonders vorteilhaft im Hinblick auf eine optimale Umformung und Färbung ist es, wenn die Zubereitungen (A) und (B) in einem Mengenverhältnis (A)/(B) von 3:1 bis 1:3, bevorzugt von 2:1 bis 1:2 und besonders bevorzugt von 1,5:1 bis 1:1,5 vermischt werden.

In einer besonders bevorzugten Ausführungsform ist ein erfindungsgemäßes Verfahren daher dadurch gekennzeichnet, dass in Schritt (I) die Zubereitungen (A) und (B) in einem Mengenverhältnis (A)/(B) von 3:1 bis 1:3, bevorzugt von 2:1 bis 1:2 und besonders bevorzugt von 1,5:1 bis 1:1,5 vermischt werden.

Während der Einwirkzeit des anwendungsbereiten Mittels (d.h. der Mischung aus den Zubereitungen (A) und (B)) auf die Faser oder bei der mechanischen Umformung kann es vorteilhaft sein, den Umformungs- und Färbevorgang durch Wärmezufuhr zu unterstützen. Die Wärmezufuhr kann durch eine externe Wärmequelle, wie z.B. warme Luft eines Warmluftgebläses, als auch, insbesondere bei einer Anwendung am lebenden Probanden, durch die Körpertemperatur des Probanden erfolgen. Bei letzterer Möglichkeit wird üblicherweise die behandelte Partie mit einer Haube abgedeckt. Eine Einwirkphase bzw. mechanische Umformung bei Raumtemperatur ist ebenfalls erfindungsgemäß. Insbesondere liegt die Temperatur während der Einwirkzeit bzw. während der Umformung zwischen 20 °C und 80 °C, insbesondere zwischen 30 °C und 65 °C. Eine Erhöhung der Temperatur über 65 °C, insbesondere über 80 °C, ist zur Vermeidung von Haarschädigungen nicht von Vorteil.

In einer weiteren besonders bevorzugten Ausführungsform ist ein erfindungsgemäßes Verfahren daher dadurch gekennzeichnet, dass in Schritt (III) die mechanische Umformung der Haare bei einer Temperatur von 20 bis 80 °C, bevorzugt bei einer Temperatur von 30 bis 65 °C erfolgt

Die in Schritt (III) des erfindungsgemäßen Verfahrens durchgeführte mechanische Umformung kann eine Wellung oder Lockung der Haare sein, diese wird beispielsweise dadurch erzielt, dass die Haare vor Anwendung des Mittels auf Wickler oder Papilloten gewickelt worden sind und das Mittel nun auf die auf den Wicklern befindlichen Haare einwirkt. Bevorzugt ist es jedoch, wenn es sich bei der mechanischen Umformung um eine Glättung der Haare handelt, die beispielsweise durch Kämmen bwz. Glatt ziehen der Haare mit einem Kamm oder einer Bürste erfolgen kann.

In einer weiteren besonders bevorzugten Ausführungsform ist ein erfindungsgemäßes Verfahren daher dadurch gekennzeichnet, dass in Schritt (III) die mechanische Umformung eine Glättung der Haare ist, die durch Kämmen bzw. Glatt ziehen der Haare mit einem Kamm oder mit einer Bürste erfolgt.

Das anwendungsbereite Umformungs- und Färbemittel kann für einen Zeitraum von 5 bis 60 Minuten, bevorzugt für 10 bis 55 Minuten und besonders bevorzugt für 25 bis 50 Minuten auf den Haaren angewendet werden.

Nach Ende der Einwirkzeit bzw. nach Abschluss des mechanischen Umformens wird das Umformungs- und Färbemittels in Schritt (IV) mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Umformungs- und Färbemittel einen stark tensidhaltigen Träger besitzt.

Optional kann das Haar danach in Schritt (V) - entweder bei Raumtemperatur oder unter Zuhilfenahme einer Wärmehaube oder eines Föhns - getrocknet werden

Das erfindungsgemäße Verfahren ist gekennzeichnet durch die Schritte (I) bis (IV), sowie durch den optionalen Schritt (V). Die genaue Abfolge der Schritte kann hierbei je nach Wunsch des Anwenders variiert werden.

In einer besonders bevorzugten Ausführungsform ist ein erfindungsgemäßes Verfahren dadurch gekennzeichnet, dass die Abfolge der Schritte im Verfahren die folgende ist: Schritt (I), gefolgt von Schritt (II), gefolgt von Schritt (III), gefolgt von Schritt (IV), gefolgt von Schritt (V)

Im Rahmen dieser Ausführungsform ist ein erfindungsgemäßes Verfahren daher gekennzeichnet durch die Abfolge der Schritte in der angegebenen Reihenfolge:
(I) Herstellung eines anwendungsbereiten Umformungs- und Färbemittels durch Vermischen einer Zubereitung (A) mit einer Zubereitung (B), wobei die Zubereitungen (A) und (B) bei der Beschreibung des ersten Erfindungsgegenstands im Detail offenbart sind,
(II) Applikation des in Schritt (I) hergestellten anwendungsbereiten Umformungs- und Färbemittel auf die Haare,
(III) mechanische Umformung der Haare, die noch mit dem anwendungsbereiten Umformungs- und Färbemittels beaufschlagt sind,
(IV) Spülen der Haare,
(V) gegebenenfalls trocknen der Haare.

In einer ebenfalls besonders bevorzugten Ausführungsform ist ein erfindungsgemäßes Verfahren dadurch gekennzeichnet, dass die Abfolge der Schritte im Verfahren die folgende ist: Schritt (I), gefolgt von Schritt (II), gefolgt von Schritt (IV), gefolgt von Schritt (III), gefolgt von Schritt (V).

Im Rahmen dieser zweiten ebenfalls besonders bevorzugten Ausführungsform ist ein erfindungsgemäßes Verfahren daher gekennzeichnet durch die Abfolge der Schritte in der angegebenen Reihenfolge:
(I) Herstellung eines anwendungsbereiten Umformungs- und Färbemittels durch Vermischen einer Zubereitung (A) mit einer Zubereitung (B), wobei die Zubereitungen (A) und (B) bei der Beschreibung des ersten Erfindungsgegenstands im Detail offenbart sind,
(II) Applikation des in Schritt (I) hergestellten anwendungsbereiten Umformungs- und Färbemittel auf die Haare,
(IV) Spülen der Haare,
(III) mechanische Umformung der Haare
(V) gegebenenfalls trocknen der Haare.

Diese zweite Ausführungsform ist für den Anwender von höherem Komfort, da die mechanische Glättung (beispielsweise durch Kämmen bzw. Glatt ziehen mit einem Kamm oder einer Bürste) auch erfolgen kann, nachdem das anwendungsbereite Färbe- und Umformungsmittel aus dem Haar wieder ausgespült wurde.

Im Rahmen dieser Ausführungsform konnte festgestellt werden, dass die in das Haar hinein diffundierten und innerhalb der Faser im Überschuss vorhandenen Resorcinderivate (A1) auch noch eine Glättung des Haares hervorrufen, wenn das Umformungs- und Färbemittel selbst bereits wieder von den Haaren abgespült worden war.

Die zuvor bei Beschreibung des ersten Erfindungsgegenstands detailliert offenbarten Zubereitungen (A) und (B) eignen sich hervorragend zur gleichzeitigen Glättung und Färbung von keratinischen Fasern.

Ein weiterer Gegenstand der vorliegenden Erfindung ist daher die Verwendung eines Mittels, das durch Vermischen von zwei getrennt konfektionierten Zubereitungen (A) und (B) hergestellt wird, zum Umformen und Färben von menschlichen Haaren, wobei die Zubereitungen (A) und (B) im Detail bei Beschreibung des ersten Erfindungsgegenstand offenbart worden sind.

Bezüglich weiterer bevorzugter Ausführungsformen des erfindungsgemäßen Verfahrens und der erfindungsgemäßen Verwendungen gilt mutatis mutandis das zu den Mehrkomponenten-Verpackungseinheiten (Kits-of-Parts) Gesagte.

### Beispiele:

### 1. Formulierungen

Es wurden die folgenden Formulierungen hergestellt - alle Angaben erfolgen, sofern nicht anders angegeben, in Gewichtsprozent Aktivsubstanz.

**Zubereitung (A)**

| | V | E |
|---|---|---|
| Cetetarylalkohol (C16-C18-Fettalkohole) | 8,5 | 8,5 |
| C12-C18-Fettalkohole | 2,0 | 2,0 |
| Texapon NSO (Laurylethersulfat, Natriumsulfat, 27,5 %ige Lösung in Wasser, INCI: Sodium Laureth-Sulfat) | 20,0 | 20,0 |
| Cocoamidopropylbetain (30 %ige Lösung in Wasser) | 12,5 | 12,5 |
| Ceteareth-20 | 0,75 | 0,75 |
| Ammoniumsulfat | 1,0 | 1,0 |
| Resorcin (A1) | -- | 10,00 |
| | | (90 mmol) |
| p-Toluylendiamin Sulfat (A2) | -- | 1,10 |
| | | (3 mmol |
| Molverhältnis (A1)/(A2) | -- | 30 |
| Ammoniak | ad pH 8,5 | ad pH 8,5 |
| Wasser | ad 100 | ad 100 |

**Zubereitungen (B)**

| | OX |
|---|---|
| Wasserstoffperoxid | 3,0 |
| Wasser | ad 100 |

Die Zubereitung (A) wurde jeweils mit der Zubereitungen (B) im Mengenverhältnis 1:1 vermischt. Bei den Strähnen, die in diesen Versuchen eingesetzt wurden, handelte es sich um gelockte Haarsträhnen gleicher Länge. Jedes dieser anwendungsbereiten Mittel wurde jeweils auf eine gelockte Haarsträhne (Kerling 6-0) appliziert (4 g Färbemittel pro 1 g Haar), dort für 45 Minuten belassen und währenddessen mehrmals mit einem Kamm glatt gezogen. Dann wurde die Strähne mit warmem Wasser gespült, nochmals mit einem Kamm glatt gekämmt und mit einem Föhn getrocknet.

Nach dem Trocknen wurde jede Strähne vor dem Hintergrund einer skalierten Schablone begutachtet, anhand der Skalierung wurde die Länge der Haarsträhne bestimmt.

Anschließend wurden die Strähnen mit einem Shampoo gewaschen, und in einer Klimakammer hängend getrocknet. Nach dem Trocknen wurden die Strähnen erneut vor dem Hintergrund einer skalierten Schablone begutachtet, und anhand der Skalierung wurde wieder die Länge der Haarsträhne bestimmt.

Je länger die Haarsträhne ist, desto weniger ist sie gelockt und desto glatter ist die Strähne.

| Zubereitung (A) + Zubereitung (B) | V + OX | E + OX |
|---|---|---|
| Länge vor dem Waschen | 15,0 cm | 15,0 cm |
| Länge nach dem Waschen | 13,5 cm | 15,0 cm |
| Bewertung der Glättung | + | +++ |

| | | |
|---|---|---|
| + = schlecht ++ = mittel +++ = gut | | |

## Patentansprüche

1. Verfahren zum Umformen und Färben von menschlichen Haaren, umfassend die folgenden Schritte:
(I) Herstellung eines anwendungsbereiten Umformungs- und Färbemittels durch Vermischen einer Zubereitung (A) mit einer Zubereitung (B),
(II) Applikation des in Schritt (I) hergestellten anwendungsbereiten Umformungs- und Färbemittels auf die Haare,
(III) mechanische Umformung der Haare,
(IV) Spülen der Haare,
(V) gegebenenfalls trocknen der Haare, wobei
- die erste Zubereitung (A) in einem kosmetischen Träger
(A1) ein oder mehrere Resorcinderivate aus der Gruppe aus Resorcin. 2-Methylresorcin und 4-Chlorresorcin, und
(A2) ein oder mehrere Oxidationsfarbstoffvorprodukte aus der Gruppe der p-Phenvlendiamine, der p-Aminophenole, der 2,4,5,6-Tetraamiopyrimidine, der 4,5-Diaminopvrazole und oder deren physiologisch verträglichen Salzen enthält, und
- die zweite Zubereitung (B) in einem kosmetischen Träger
(B1) Wasserstoffperoxid enthält,
**dadurch gekennzeichnet, dass** das molare Verhältnis aus allen in der Zubereitung (A) enthaltenen Resorcinderivaten (A1) zu allen in der Zubereitung (A) enthaltenen Oxidationsfarbstoffen vom Entwicklertyp (A2), d.h. das molare Verhältnis (A1)/(A2), bei einem Wert von mindestens 1,2 liegt.

2. Verfahren nach Anspruch 1, **dadurch gekennzeichnet, dass**
- die erste Zubereitung (A) - bezogen auf das Gesamtgewicht der Zubereitung (A) - (A1) ein oder mehrere Resorcinderivate aus der Gruppe aus Resorcin. 2-Methvlresorcin und 4-Chlorresorcin in einer Gesamtmenge von 1.0 bis 15.0 Gew-%, bevorzugt von 2,0 bis 13,0 Gew.-%, weiter bevorzugt von 3,0 bis 12,0 Gew.-%, noch weiter bevorzugt von 4,0 bis 11,0 Gew.-% und ganz besonders bevorzugt von 5.0 bis 10,5 Gew.-% enthält.

3. Verfahren nach einem der Ansprüche 1 bis 2, **dadurch gekennzeichnet, dass**
- die erste Zubereitung (A)
(A2) einen oder mehrere Oxidationsfarbstoffvorprodukte vom Entwicklertyp enthält, die ausgewählt sind aus der Gruppe aus p-Phenylendiamin, p-Toluylendiamin, 2-(2-Hydroxvethyl)-p-phenylendiamin, N, N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)methan, p-Aminophenol, 4-Amino-3-methylphenol, 4,5-Diamino-1-(2-hydroxyethyl)pyrazol, 2,4,5,6-Tetraaminopyrimidin und/oder deren physiologisch verträglichen Salzen.

4. Verfahren nach einem der Ansprüche 1 bis 3, **dadurch gekennzeichnet**,
- die erste Zubereitung (A) - bezogen auf das Gesamtgewicht der Zubereitung (A) - (A2) einen oder mehrere Oxidationsfarbstoffvorprodukte vom Entwicklertyp in einer Gesamtmenge von 0,1 bis 2,5 Gew.-%, bevorzugt von 0,2 bis 2,3 Gew.-%, weiter bevorzugt von 0,6 bis 1,7 Gew.-% und besonders bevorzugt von 0,9 bis 1,1 Gew.-% enthält.

5. Verfahren nach einem der Ansprüche 1 bis 4, **dadurch gekennzeichnet, dass** das molare Verhältnis aus allen in der Zubereitung (A) enthaltenen Resorcinderivaten (A1) zu allen in der Zubereitung (A) enthaltenen Oxidationsfarbstoffvorprodukten vom Entwicklertyp (A2), d.h. das molare Verhältnis (A1)/(A2), bei einem Wert von 1,3 bis 60,0, bevorzugt von 1,5 bis 50,0, weiter bevorzugt von 2,0 bis 45,0, noch weiter bevorzugt von 4.0 bis 35,0 und besonders bevorzugt von 7.0 bis 32,0 liegt.

6. Verfahren nach einem der Ansprüche 1 bis 5, **dadurch gekennzeichnet, dass**
- die zweite Zubereitung (B) - bezogen auf das Gesamtgewicht der Zubereitung (B) - Wasserstoffperoxid in einer Menge von 0,1 bis 12,0 Gew.-%, bevorzugt von 0,15 bis 5,0 Gew.-%, weiter bevorzugt von 0,2 bis 4,5 Gew.-% und ganz besonders bevorzugt von 0,3 bis 4,2 Gew.-% enthält.

7. Verfahren nach einem der Ansprüche 1 bis 6, **dadurch gekennzeichnet, dass** die erste Zubereitung (A) - bezogen auf das Gesamtgewicht der Zubereitung (A) - reduzierende Substanzen aus der Gruppe aus Thioglykolsäure, Thiomilchsäure, Cystein und den Salzen dieser Verbindungen in einer Gesamtmenge von weniger als 1,0 Gew.-%, bevorzugt von weniger als 0,5 Gew.-%, weiter bevorzugt von weniger als 0.1 Gew.-% und besonders bevorzugt von weniger als 0,05 Gew.-% enthält.

8. Verfahren nach einem der Ansprüche 1 bis 7, **dadurch gekennzeichnet, dass** die Zubereitung (A) und/oder die Zubereitung (B) mindestens ein Quellmittel, bevorzugt aus der Gruppe aus Harnstoff (Urea), Glycerin (Propan-1,2,3-triol), Sorbit (D-Glucit) und/oder Pyrrolidoncarbonsäure (5-Oxopyrrolidin-2-carbonsäure) enthält.

9. Verfahren nach einem der Ansprüche 1 bis 8, **dadurch gekennzeichnet, dass** in Schritt (I) die Zubereitungen (A) und (B) in einem Mengenverhältnis (A)/(B) von 3:1 bis 1:3, bevorzugt von 2:1 bis 1:2 und besonders bevorzugt von 1,5:1 bis 1:1,5 vermischt werden.

10. Verfahren nach einem der Ansprüche 1 bis 9, **dadurch gekennzeichnet, dass** in Schritt (III) die mechanische Umformung der Haare bei einer Temperatur von 20 bis 80 °C, bevorzugt bei einer Temperatur von 30 bis 65 °C erfolgt.

11. Verfahren nach einem der Ansprüche 1 bis 10, **dadurch gekennzeichnet, dass** in Schritt (III) die mechanische Umformung eine Glättung der Haare ist, die durch Kämmen bzw. Glattziehen der Haare mit einem Kamm oder mit einer Bürste erfolgt.

12. Verfahren nach einem der Ansprüche 1 bis 11, **dadurch gekennzeichnet, dass** die Abfolge der Schritte im Verfahren die folgende ist: Schritt (I), gefolgt von Schritt (II), gefolgt von Schritt (III), gefolgt von Schritt (IV), gefolgt von Schritt (V).

13. Verfahren nach einem der Ansprüche 1 bis 12, **dadurch gekennzeichnet, dass** die Abfolge der Schritte im Verfahren die folgende ist: Schritt (I), gefolgt von Schritt (II), gefolgt von Schritt (IV), gefolgt von Schritt (III), gefolgt von Schritt (V).

14. Verwendung eines Mittels, das durch Vermischen von zwei getrennt konfektionierten Zubereitungen (A) und (B) hergestellt wird, zum Umformen und Färben von menschlichen Haaren, wobei die Zubereitungen (A) und (B) wie in den Ansprüchen 1 bis 8 definiert sind.

## Claims

1. A method for shaping and coloring human hair, comprising the following steps:
(I) preparing a ready-to-use shaping and coloring agent by mixing a preparation (A) with a preparation (B),
(II) applying the ready-to-use shaping and coloring agent prepared in step (I) to the hair,
(III) mechanically shaping the hair,
(IV) rinsing the hair,
(V) optionally drying the hair,
- the first preparation (A) containing, in a cosmetic carrier,
(A1) one or more resorcinol derivatives from the group of resorcinol, 2-methylresorcinol and 4-chlororesorcinol, and
(A2) one or more oxidation dye precursors from the group of p-phenylenediamines, the p-aminophenols, the 2,4,5,6-tetraamiopyrimidines, the 4,5-diaminopyrazoles and/or the physiologically acceptable salts thereof, and
- the second preparation (B) containing, in a cosmetic carrier,
(B1) hydrogen peroxide,
**characterized in that** the molar ratio of all resorcinol derivatives (A1) contained in preparation (A) to all developer-type oxidation dyes (A2) contained in preparation (A), i.e. the molar ratio (A1)/(A2), is at a value of at least 1.2.

2. The method according to claim 1, **characterized in that**
- the first preparation (A) contains, based on the total weight of the preparation (A),
(A1) one or more resorcinol derivatives from the group of resorcinol, 2-methylresorcinol and 4-chlororesorcinol in a total amount of from 1.0 to 15.0 wt.%, preferably from 2.0 to 13.0 wt.%, more preferably from 3.0 to 12.0 wt.%, even more preferably from 4.0 to 11.0 wt.% and most particularly preferably from 5.0 to 10.5 wt.%.

3. The method according to one of claims 1 to 2, **characterized in that**
- the first preparation (A) contains
(A2) one or more developer-type oxidation dye precursors selected from the group of p-phenylenediamine, p-toluenediamine, 2-(2-hydroxyethyl)-p-phenylenediamine, N,N-bis-(2-hydroxyethyl)-p-phenylenediamine, 2-methoxymethyl-p-phenylenediamine, N-(4-amino-3-methylphenyl)-N-[3-(1H-imidazol-1-ylpropyl]amine, bis-(2-hydroxy-5-aminophenyl)methane, p-aminophenol, 4-amino-3-methyphenol, 4,5-diamino-1-(2-hydroxyethyl)pyrazole, 2,4,5,6-tetraaminopyrimidine and/or the physiologically acceptable salts thereof.

4. The method according to one of claims 1 to 3, **characterized in that**
- the first preparation (A) contains, based on the total weight of the preparation (A),
(A2) one or more developer-type oxidation dye precursors in a total amount of from 0.1 to 2.5 wt.%, preferably from 0.2 to 2.3 wt.%, more preferably from 0.6 to 1.7 wt.%, and particularly preferably from 0.9 to 1.1 wt.%.

5. The method according to one of claims 1 to 4, **characterized in that** the molar ratio of all resorcinol derivatives (A1) contained in the preparation (A) to all developer-type oxidation dye precursors (A2) contained in the preparation (A), i.e. the molar ratio (A1)/(A2), is at a value of from 1.3 to 60.0, preferably from 1.5 to 50.0, more preferably from 2.0 to 45.0, even more preferably from 4.0 to 35.0 and most particularly preferably from 7.0 to 32.0.

6. The method according to one of claims 1 to 5, **characterized in that**
- the second preparation (B) contains, based on the total weight of the preparation (B), hydrogen peroxide in an amount of from 0.1 to 12.0 wt.%, preferably from 0.15 to 5.0 wt.%, more preferably from 0.2 to 4.5 wt.% and very particularly preferably from 0.3 to 4.2 wt.%.

7. The method according to one of claims 1 to 6, **characterized in that** the first preparation (A) contains, based on the total weight of the preparation (A), reducing substances from the group of thioglycolic acid, thiolactic acid, cysteine and the salts of these compounds in a total amount of less than 1.0 wt.%, preferably less than 0.5 wt.%, more preferably less than 0.1 wt.% and particularly preferably less than 0.05 wt.%.

8. The method according to one of claims 1 to 7, **characterized in that** the preparation (A) and/or the preparation (B) contains at least one swelling agent, preferably from the group of urea, glycerol (propane-1,2,3-triol), sorbitol (d-glucitol) and/or pyrrolidone carboxylic acid (5-oxopyrrolidine-2-carboxylic acid).

9. The method according to one of claims 1 to 8, **characterized in that** in step (I) the preparations (A) and (B) are mixed in a ratio (A)/(B) of 3:1 to 1:3, preferably from 2:1 to 1:2 and particularly preferably from 1.5:1 to 1:1.5.

10. The method according to one of claims 1 to 9, **characterized in that** in step (III) the hair is mechanically shaped at a temperature of from 20 to 80 °C, preferably at a temperature of from 30 to 65 °C.

11. The method according to one of claims 1 to 10, **characterized in that** in step (III) the mechanical shaping is a smoothing of the hair, which is carried out by combing or straightening the hair using a comb or using a brush.

12. The method according to one of claims 1 to 11, **characterized in that** the sequence of steps in the method is the following: step (I), followed by step (II), followed by step (III), followed by step (IV), followed by step (V).

13. The method according to one of claims 1 to 12, **characterized in that** the sequence of steps in the method is the following: step (I), followed by step (II), followed by step (IV), followed by step (III), followed by step (V).

14. The use of an agent which is prepared by mixing two separately prepared preparations (A) and (B) for shaping and coloring human hair, wherein the preparations (A) and (B) are defined as in claims 1 to 8.

## Revendications

1. Procédé de mise en forme et de coloration des cheveux humains, comprenant les étapes suivantes :
(I) préparation d'un agent de mise en forme et de coloration prêt à l'emploi par mélange d'une préparation (A) avec une préparation (B),
(II) application, sur les cheveux, de l'agent de mise en forme et de coloration prêt à l'emploi préparé à l'étape (I),
(III) mise en forme mécanique des cheveux,
(IV) rinçage des cheveux,
(V) éventuellement, séchage des cheveux,
- la première préparation (A) contenant, dans un support cosmétique,
(A1) un ou plusieurs dérivés de résorcinol du groupe du résorcinol, du 2-méthylrésorcinol et du 4-chlororésorcinol, et
(A2) un ou plusieurs précurseurs de colorants d'oxydation du groupe des p-phénylènediamines. des p-aminophénols, des 2,4,5,6-tétraamiopyrimidines, des 4,5-diaminopyrazoles et/ou de leurs sels physiologiquement acceptables, et
- la seconde préparation (B) contenant, dans un support cosmétique,
(B1) du peroxyde d'hydrogène,
**caractérisé en ce que** le rapport molaire entre tous les dérivés de résorcinol (A1) contenus dans la préparation (A) et tous les colorants d'oxydation de type développeur (A2) contenus dans la préparation (A), c'est-à-dire le rapport molaire (A1)/(A2), est d'une valeur d'au moins 1,2.

2. Procédé selon la revendication 1, **caractérisé en ce que**
- la première préparation (A) contient - sur la base du poids total de la préparation (A) -
(A1) un ou plusieurs dérivés de résorcinol du groupe du résorcinol, du 2-méthylrésorcinol et du 4-chlororésorcinol en une quantité totale de 1,0 à 15,0 % en poids, préférentiellement de 2,0 à 13,0 % en poids, plus préférentiellement de 3,0 à 12,0 % en poids, encore plus préférentiellement de 4,0 à 11,0 % en poids et de manière tout particulièrement préférée de 5,0 à 10,5 % en poids.

3. Procédé selon l'une des revendications 1 à 2, **caractérisé en ce que**
- la première préparation (A)
(A2) contient un ou plusieurs précurseurs de colorants d'oxydation de type développeur choisis dans le groupe de la p-phénylènediamine, de la p-toluènediamine, de la 2-(2-hvdroxvéthyl)-p-phénvlènediamine, de la N,N-bis-(2-hvdroxvéthyl)-p-phénylène diamine, de la 2-méthoxyméthyl-p-phénylènediamine, de la N-(4-amino-3-méthylphényl)-N-[3-(1H-imidazol-1-ylpropyllamine, du bis-(2-hvdroxv-5-aminophényl)méthane, du p-aminophénol, du 4-amino-3-méthyphénol, du 4,5-diamino-1-(2-hydroxyéthyl)pvrazole, de la 2,4,5,6-tétraaminopvrimidine et/ou de leurs sels physiologiquement acceptables.

4. Procédé selon l'une des revendications 1 à 3, **caractérisé en ce que**
- la première préparation (A) contient - sur la base du poids total de la préparation (A) -
(A2) un ou plusieurs précurseurs de colorants d'oxydation de type développeur en une quantité totale de 0,1 à 2,5 % en poids, préférentiellement de 0,2 à 2,3 % en poids, plus préférentiellement de 0,6 à 1,7 % en poids et de manière particulièrement préférée de 0,9 à 1,1 % en poids.

5. Procédé selon l'une des revendications 1 à 4, **caractérisé en ce que** le rapport molaire entre tous les dérivés de résorcinol (A1) contenus dans la préparation (A) et tous les précurseurs de colorants d'oxydation de type développeur (A2) contenus dans la préparation (A), c'est-à-dire le rapport molaire (A1)/(A2), est d'une valeur de 1,3 à 60,0, préférentiellement de 1,5 à 50,0, plus préférentiellement de 2,0 à 45,0, encore plus préférentiellement de 4,0 à 35,0 et de manière particulièrement préférée de 7,0 à 32,0.

6. Procédé selon l'une des revendications 1 à 5, **caractérisé en ce que**
- la seconde préparation (B) contient - sur la base du poids total de la préparation (B) - du peroxyde d'hydrogène en une quantité de 0,1 à 12,0 % en poids, préférentiellement de 0,15 à 5,0 % en poids, plus préférentiellement de 0,2 à 4,5 % en poids et de manière tout particulièrement préférée de 0,3 à 4,2 % en poids.

7. Procédé selon l'une des revendications 1 à 6, **caractérisé en ce que** la première préparation (A) contient - sur la base du poids total de la préparation (A) - des substances réductrices du groupe de l'acide thioglycolique, de l'acide thiolactique, de la cystéine et des sels de ces composés en une quantité totale inférieure à 1,0 % en poids, préférentiellement inférieure à 0,5 % en poids, plus préférentiellement inférieure à 0,1 % en poids et de manière particulièrement préférée inférieure à 0,05 % en poids.

8. Procédé selon l'une des revendications 1 à 7, **caractérisé en ce que** la préparation (A) et/ou la préparation (B) contiennent au moins un agent gonflant, préférentiellement issu du groupe constitué par l'urée (Urea), le glycérol (propane-1,2,3-triol), le sorbitol (D-glucite) et/ou l'acide pyrrolidone carboxylique (acide 5-oxopyrrolidine-2-carboxylique).

9. Procédé selon l'une des revendications 1 à 8, **caractérisé en ce qu'**à l'étape (I), les préparations (A) et (B) sont mélangées dans un rapport de quantités (A)/(B) de 3:1 à 1:3, préférentiellement de 2:1 à 1:2 et de manière particulièrement préférée de 1,5:1 à 1:1,5.

10. Procédé selon l'une des revendications 1 à 9, **caractérisé en ce qu'**à l'étape (III), la mise en forme mécanique des cheveux est effectuée à une température de 20 à 80 °C, préférentiellement à une température de 30 à 65 °C.

11. Procédé selon l'une des revendications 1 à 10, **caractérisé en ce qu'**à l'étape (III), la mise en forme mécanique est un lissage des cheveux, lequel est effectué par peignage ou lissage des cheveux avec un peigne ou avec une brosse.

12. Procédé selon l'une des revendications 1 à 11, **caractérisé en ce que** la séquence des étapes du procédé est la suivante : l'étape (I) suivie de l'étape (II) suivie de l'étape (III) suivie de l'étape (IV) suivie de l'étape (V).

13. Procédé selon l'une des revendications 1 à 12, **caractérisé en ce que** la séquence des étapes du procédé est la suivante : l'étape (I) suivie de l'étape (II) suivie de l'étape (IV) suivie de l'étape (III) suivie de l'étape (V).

14. Utilisation d'un agent qui est préparé par mélange de deux préparations (A) et (B) conditionnées séparément pour la mise en forme et la coloration des cheveux humains, les préparations (A) et (B) étant définies telles que dans les revendications 1 à 8.
